(19) Europäisches Patentamt
European Patent Office
Office européen des brevets

(11) Publication number : 0 423 293 B1

# (12) EUROPEAN PATENT SPECIFICATION

(45) Date of publication of patent specification : 21.04.93 Bulletin 93/16

(51) Int. Cl.$^5$ : A61K 7/02

(21) Application number : 90906953.6

(22) Date of filing : 30.04.90

(86) International application number : PCT/EP90/00694

(87) International publication number : WO 90/13282 15.11.90 Gazette 90/26

## (54) DECORATIVE COMPOSITION.

(30) Priority : 11.05.89 GB 8910803

(43) Date of publication of application : 24.04.91 Bulletin 91/17

(45) Publication of the grant of the patent : 21.04.93 Bulletin 93/16

(84) Designated Contracting States : CH DE FR GB LI

(56) References cited :
DE-A- 3 212 420
US-A- 4 301 023
Patent Abstracts of Japan, vol. 8, no. 77 (C-218)(1514), 10 April 1984 & JP-A-591407 (Kao Sekken K.K.), 6 January 1984

(73) Proprietor : MERCK PATENT GESELLSCHAFT MIT BESCHRÄNKTER HAFTUNG
Frankfurter Strasse 250
W-6100 Darmstadt (DE)

(72) Inventor : SAGE, Ian, Charles
58 Wentworth Drive Broadstone
Dorset BH18 8EG (GB)

EP 0 423 293 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The application relates to the use of a decorative composition comprising in admixture:

a) one or more non-chiral compound of the formula I

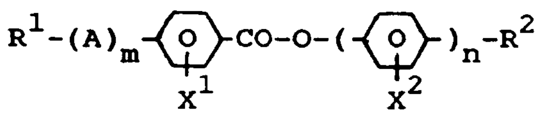

I

wherein

$R^1$ and $R^2$ are each independently a normal or branched alkyl or alkenyl residue with up to 16 C atoms wherein one or two nonadjacent $CH_2$ groups of these residues may be replaced by -O- or -CO-

A denotes a group of the formula

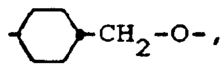 , 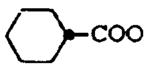 , or 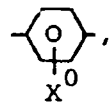 ,

$X^0$, $X^1$ and $X^2$ are each independently hydrogen or an halogen atom,

m is 0 or 1, and

n is 1 or 2,

b) one or more chiral steroid ester of the formula II

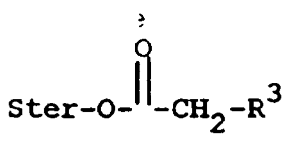

II

wherein

$R^3$ is a normal or branched alkyl or alkenyl residue with up to 16 C atoms wherein one $CH_2$ group may be replaced by -O-, -O-CO- or -CO-O-, and

Ster denotes a saturated or unsaturated gonan-3-yl group being optionally substituted by up to 6 normal or branched alkyl residues with 1 to 10 C atoms, and

c) at least one vehicle and, if desired, at least one auxiliary.

for decorative applications.

For decorative applications, particularly in cosmetics there is a need for new colour effects which impart to the formulations an advantageous appearance and contain physiologically acceptable dyestuffs permitted in cosmetics.

However, in addition to the coloured gloss and the aesthetic effects achieved with this, the behaviour of the formulation on the skin, the so-called "feeling", also plays a decisive role in cosmetic formulations. The known cosmetic pigments are still in need of improvement in this respect.

It is known that compositions containing p-alkyl-(or p-alkoxy)phenyl p-alkyl-(or p-alkoxy)benzoates and an optically active cholesteryl ester are suitable for temperature indicating devices, the British Patent Specification 1583137 and 1583137 describe compositions containing, for example, cholesteryl oleate, p-pentylphenyl-p-pentyloxybenzoate and p-pentylphenyl-p-methoxybenzoate and being useful as temperature indicating compositions. But there is no hint that such compositions can be used as decorative compositions.

It is now been found, surprisingly, that when decorative compositions being an admixture of compounds of the formula I, chiral steroid esters of the formula II, vehicles and optionally auxiliaries are used in cosmetic formulations, particularly skin-friendly pleasant preparations are obtained, and very aesthetic effects can also be achieved by the metallic coloured gloss.

The invention therefore relates to the use of a decorative composition comprising an admixture of at least one non-chiral compound of the formula I, at least one chiral steroid ester of the formula II and at least one vehicle and, if desired, at least one auxiliary for decorative applications.

Liquid crystal phases are exhibited by certain organic compounds and constitute an intermediate state

which exists between the crystalline solid and the fully disordered liquid phase and within which certain long range ordering of the molecules takes place.

There are two important types of liquid crystal phase; the smectic mesophase in which the long range ordering is of a substantially lamellar type and the nematic mesophase in which the ordering is substantially linear, i.e. the molecules tend to line up with the long axes of the molecules parallel.

Included sometimes as a subclass of the nematic mesophase and sometimes classified as a separate mesophase is the cholesteric mesophase. This last has a helical long range order imposed upon the linear order of the nematic mesophase. Compounds displaying a cholesteric mesophase are optically active (chiral) and the pitch of the helical twist is determined by the nature and extent of the optical activity. The pitch of the helical twist may be such that thin films of the cholesteric phase reflect visible light, resulting in the observation of bright colours.

The chiral steroid esters of the formula II induce a cholesteric mesophase (hereinafter designated Ch) because of their molecular shape and optical activity to the liquid crystalline composition confining the compounds of the formula I which possess a nematic mesophase. As a result of these properties, the compositions according to the invention have not only a high refractive index and high gloss but also a very good skin feeling.

The compounds of the formula I used according to the invention are known per se and have also already been proposed as components for liquid crystalline compositions which can be applied in electro-optical display devices or in temperature indicating devices. Processes for their preparation are described, for example, by R. Steinsträsser, Z. Naturforsch. 27b, 774 (1972), German Patent Application 2240864, U.S. Patent 4,136,053 or the British Patent Applications 8800800, 8800801 and 88 11 374 and other publications quoted in the literature mentioned above.

Above and below $R^1$, $R^2$, $R^3$, A, $X^0$, $X^1$ and $X^2$, m and n have the meaning given, unless expressly indicated otherwise.

Phe denotes a 1,4-phenylen group, PheX denotes a 1,4-phenylen group substitued by an halogen atom and Cy denotes a trans-1,4-cyclohexylen group.

The compounds of the formula I accordingly include preferred compounds with two rings of the part formulae Ia to Id:

$R^1$-Phe-COO-Phe-$R^2$ Ia

$R^1$-PheX-COO-Phe-$R^2$ Ib

$R^1$-Phe-COO-Phe-$R^2$ Ic

$R^1$-PheX-COO-PheX-$R^2$ Id

compounds with three rings of the part formulae Ie to Il

$R^1$-PhePhe-COO-Phe-$R^2$ Ie

$R^1$-PheX-Phe-COO-Phe-$R^2$ If

$R^1$-Phe-Phe-COO-PheX-$R^2$ Ig

$R^1$-Phe-COO-PhePhe-$R^2$ Ih

$R^1$-Cyc-$CH_2$O-Phe-COO-Phe-$R^1$ Ii

$R^1$-Cyc-COO-Phe-COO-Phe-$R^2$ Ij

$R^1$-Cyc-$CH_2$O-PheX-COO-Phe-$R^2$ Ik

$R^1$Cyc-$CH_2$O-Phe-COO-PheX-$R^2$ Il

and compounds with four rings of the part formulae Im to Io

$R^1$-PhePhe-COO-PhePhe-$R^2$ Im

$R^1$-Cyc-COO-Phe-COO-PhePhe-$R^2$ In

$R^1$-Cyc-$CH_2$O-Phe-COO-PhePhe-$R^2$ Io

Above and below $R^1$ und $R^2$ denote preferably alkyl or alkoxy. In the compounds of the formula PheX denotes a 1,4-phenylen group substituted by an halogen atom, preferably by fluorine or alkoxy.

n preferabls is 1 or 2, particularly 1.

m preferabls is O.

Above and below $R^1$ and $R^2$ each preferably denote alkyl with preferably 1 to 13 C atoms, particularly 3 to 12 C atoms wherein one or the two $CH_2$ groups may be replaced by -O- or -CO-.

Preferably only one $CH_2$ group is replaced, particularly preferred by -O-.

If $R^1$ and $R^2$ each are an alkyl radical wherein one ("alkoxy" or "oxaalkyl") or two ("alkoxyalkoxy" or "dioxaalkyl") nonadjacent $CH_2$ groups may be replaced by -O-, these radicals can be straight-chain or branched. Preferably, it is straight-chain and has 2, 3, 4, 5, 6, 7, 8, 9 or 10 C atoms and is accordingly preferably ethyl, propyl, butyl, pentyl, hexyl, heptyl, octyl, nonyl or decyl, ethoxy, propoxy, butoxy, penyloxy, hexyloxy, heptyloxy, nonyloxy, decyloxy, also methyl, undecyl, dodecyl, tridecyl, tetradecyl, methoxy, undecyloxy, dodecyloxy, tridecyloxy or tetradecyloxy.

Oxaalkyl is preferably straight chain 2-oxapropyl (= methoxymethyl), 2-(=ethoxymethyl) or 3-oxabutyl (=

2-methoxyethyl), 2-, 3- or 4-oxapentyl, 2-, 3-, 4- or 5-oxahexyl, 2-, 3-, 4-, 5- or 6-oxaheptyl, 2-, 3-, 4-, 5-, 6- or 7-oxaoctyl, 2-, 3-, 4-, 5-, 6-, 7- or 8-oxanonyl, 2-, 3-, 4-, 5-, 6-, 7-, 8- or 9-oxadecyl, 1,3-dioxabutyl (= methoxymethoxy), 1,3-, 1,4-, 2,4-dioxapentyl, 1,3-, 1,4-, 1,5-, 2,4-, 2,5- or 3,5-dioxahexyl, 1,3-, 1,4-, 1,5-, 1,6-, 2,4-, 2,5-, 2,6-, 3,5-, 3,6- or 4,6-dioxaheptyl.

The chiral steroid esters of the formula II used according the invention are also known per se, for example Flüssigkristalle in Tabellen, VEB Deutscher Verlag für Grundstoff-industrie, Leipzig Vol. I 1974 and Vol. II, 1984.

In the compounds of the formula II Ster denotes a saturated or unsaturated gonan-3-yl group of the formula III

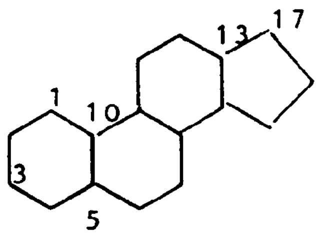

III

being preferably substituted in the 5-, 10-, 13- and/or 17-position by normal or branched alkyl residues with up to 10 C-atoms.

The compounds of the formula II wherein Ster denotes a cholesterin-3-yl group are preferred.

Furthermore preferred are those compounds of the formula II wherein Ster denotes a cholestan-3-yl group, a cholest-2-en-3-yl group, a sitosterin-3-yl group, an 17-alkylandrostan-3-yl group, an 17-alkylandrosten-3-yl group or an estron-3-yl group.

The residue $R^3$ of the formula II preferably is an alkyl group with 1 to 13, preferably 2 to 10 C atoms.

The inventive cholesteric liquid crystalline phases formed by the compounds of the formulae I and II preferably contain at least one preferably two or more non-chiral compounds of the formula I and two, three or more, chiral steroid esters of the formula II. Said cholesteric liquid crystalline phases contain about 20 to 90 preferably 40 to 75 % of one or more compounds of the formula I and about 10 to 80 preferably 25 to 60 % of one or more compounds of the formula II. In these cholesteric liquid crystalline phases the amount of the compound of the formula I and the amount of the compound of the formula II add up to 100 %.

The clearing point of these cholesteric liquid crystalline phases (Ch-I) lies preferably between 37 ° and 100 °C particularly preferred between 50 °C and 90 °C.

The cholesteric liquid crystalline phases used in the present invention are prepared in a manner which is customary per se. As a rule, the components are dissolved in one another preferably at elevated temperature.

Because of the good gloss and the low weight of the cholesteric liquid crystalline phases, only relatively small amounts thereof is required in the decorative composition. Although"the absolute amount depends on the nature of the formulation and the desired coloured effect, as a rule about 5 to about 80 % by weight of the preferably 10 to 40 % are employed.

The cholesteric liquid crystalline phases may be microencapsulated before admixing to the vehicles.

The microencapsulated cholesteric liquid crystalline phases are prepared in a manner which is customary per se (For example U.S. Patent 2,800,457).

Vehicles used for the formulations according to the invention are the substances customary for, for example, lipsticks, grease sticks, creams, powders and other cosmetics. These are known to the expert or are to be found in standard works, such as, for example, H. Janistyn, Handbuch der Kosmetika and Riechstoffe (Handbook of Cosmetics and Perfumes), Hüthig Verlag Heidelberg. Preferred vehicles are, for example, water based binders like polyvinyl alcoholes or polyurethanes.

The formulations according to the invention contain as colouring constituents in each case at least one of the abovementioned cholesteric liquid crystalline phases formed by the compounds of the formula I and the compounds of the formula II.

Very attractive coloured effects are achieved. The present invention therefore provides very advantageous new decorative compositions with very attractive coloured effects and a very pleasant skin feeling.

The following examples are intended to illustrate the invention without limiting it. Percentages above and below are percentages by weight. All the temperatures are given in degrees Centigrade. The symbols are furthermore as follows: Cr: crystalline solid state, S: smectic phase (the index characterizes the phase type), N: nematic phase, Ch: cholesteric phase, I: isotropic phase. The figure between two symbols indicates the transition temperature.

Example 1

A cholesteric liquid crystalline mixture is formulated containing the following liquid crystalline components

| | |
|---|---|
| p-pentylphenyl-p-methoxybenzoate | 49.3 % |
| p-pentylphenyl-p-hexyloxybenzoate | 24.7 % |
| cholesteryl nonanoate | 11.7 % |
| cholesteryl valerate | 7.4 % |
| cholesteryl propionate | 6.9 % |

exhibits a bright, red colour and Ch 52.3 ° I

Example 2

A cholesteric liquid crystalline mixture is formulated containing the following liquid crystalline components

| | |
|---|---|
| p-pentylphenyl-p-methoxybenzoate | 46.7 % |
| p-pentylphenyl-p-hexyloxybenzoate | 23.3 % |
| cholesteryl nonanoate | 13.5 % |
| cholesteryl valerate | 8.6 % |
| cholesteryl propionate | 7.9 % |

exhibits a bright, yellow colour and Ch 53.2 ° I

Example 3

A cholesteric liquid crystalline mixture is formulated containing the following liquid crystalline components

| | |
|---|---|
| p-pentylphenyl-p-methoxybenzoate | 44.5 % |
| p-pentylphenyl-p-hexyloxybenzoate | 22.2 % |
| cholesteryl nonanoate | 15.0 % |
| cholesteryl valerate | 9.5 % |
| cholesteryl propionate | 8.8 % |

exhibits a bright, green colour and Ch 53.9 ° I

Example 4

A cholesteric liquid crystalline mixture is formulated containing the following liquid crystalline components

| | |
|---|---|
| p-pentylphenyl-p-methoxybenzoate | 41.0 % |
| p-pentylphenyl-p-hexyloxybenzoate | 20.5 % |
| cholesteryl nonanoate | 17.3 % |
| cholesteryl valerate | 11.0 % |
| cholesteryl propionate | 10.2 % |

exhibits a bright, blue colour and Ch 55.6 ° I

Example 5

A cholesteric liquid crystalline mixture is formulated containing the following liquid crystalline components

| | |
|---|---|
| p-pentylphenyl-p-methoxybenzoate | 40.0 % |
| p-pentylphenyl-p-hexyloxybenzoate | 20.0 % |
| cholesteryl nonanoate | 18.0 % |
| cholesteryl valerate | 11.1 % |
| cholesteryl propionate | 10.6 % |

exhibits a bright, blue colour and Ch 56 ° I

Example 6

A cholesteric liquid crystalline mixture is formulated containing the following liquid crystalline components

| | |
|---|---|
| p-pentylphenyl-p-methoxybenzoate | 38.0 % |
| p-pentylphenyl-p-hexyloxybenzoate | 19.0 % |
| cholesteryl nonanoate | 19.3 % |
| cholesteryl valerate | 12.3 % |
| cholesteryl propinate | 11.4 % |

exhibits a bright, purple colour and Ch 56.8 ° I

Example 7

A cholesteric liquid crystalline mixture is formulated containing the following liquid crystalline components

| | |
|---|---|
| p-pentylphenyl-p-methoxybenzoate | 18.0 % |
| p-pentylphenyl-p-hexyloxybenzoate | 16.0 % |
| p-pentylphenyl-p-octyloxybenzoate | 16.0 % |
| cholesteryl valerate | 25.0 % |
| cholesteryl nonanoate | 25.0 % |

exhibits a bright colour and Ch 57.6 ° I.

The cholesteric liquid crystalline mixtures of the examples 1 to 7 are admixed to customary vehicles to achieve decorative compositions with attractive coloured effects and pleasant skin feeling.

Example 8

Decorative cosmetic composition formulation

A solution of Carbopol 940 (BF Goodrich Co, Cleveland, Ohio) at a solution of 0.25 % in water is neutralised with vigorous stirring to a pH 7.0 by careful addition of sodium hydroxide solution. A clear highly viscous gel is formed. By use of a nozzle, strands of chiral nematic liquid crystal (of example 1) are injected into the gel to obtain an attractive cosmetic formulation having an irridescent colour effect in the bulk sample, and a pleasant smooth feel when rubbed into the skin.

Example 9

Liquid crystal mascara formulation

The chiral nematic liquid crystal mixtureof example 6 is incorporated into capsules of gelatin/gum arabic coacervate by the process of U.S. Patent 28,00,457 example 2, incorporating the optional hardening step described in example 1 of the same patent. The stirrer speed is adjusted to provide capsules of mean diameter 0.6 mm. The capsules are dispersed into a Carbopol gel obtained as above at a concentration of 5 % to obtain a clear mascara which imparts irridescent highlights to the eyelash.

Example 10

Irridescent decorative Coating

The chiral nematic liquid crystal mixture of example 3 is microencapsulated in a gelatin/gum arabic coacervate system to yield capsules of mean diameter 10 μm. The aquaeous capsule slurry is filtered from excess water and incorporated in a solution of polyvinyl alcohol to yield a final liquid crystal content of 20 % and PVA content of 8 %. The resulting viscous slurry is applied by screenprinting through a mesh of 70 threads per centimetre to a black board, and after drying imparts an attractive irridescent colour effect.

## Claims

**1.** Use of a decorative composition comprising in admixture:

a) one or more non-chiral compound of the formula I

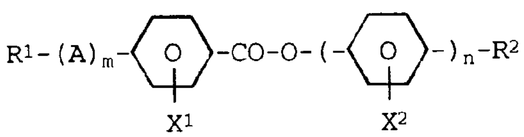 I

wherein

$R^1$ and $R^2$ are each independently a normal or branched alkyl or alkenyl residue with up to 16 C atoms wherein one or two nonadjacent $CH_2$ groups of these residues may be replaced by -O- or -CO-

A denotes a group of the formula

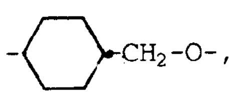 , 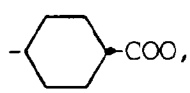 , or 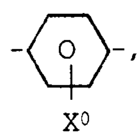 ,

$X^0$, $X^1$ and $X^2$ are each independently hydrogen or an halogen atom,

m is 0 or 1, and

n is 1 or 2,

b) one or more chiral steroid ester of the formula II

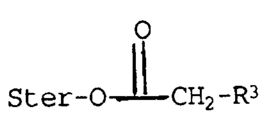 II

wherein

$R^3$ is a normal or branched alkyl or alkenyl residue with up to 16 C atoms wherein one $CH_2$ group may be replaced by -O-, -O-CO- or -CO-O-, and

Ster denotes a saturated or unsaturated gonan-3-yl group being optionally substituted by up

to 6 normal or branched alkyl residues with 1 to 10 C atoms, and
c) at least one vehicle and, if desired, at least one auxiliary
for decorative applications.

**2.** Use of a decorative composition according to Claim 1 characterized in that m is 0 and n is 1 in formula I.

**3.** Use of a decorative composition to Claim 1 or 2 characterized in that Ster denotes a cholesterin-4-yl group.

**4.** A decorative composition comprising in admixture:
a) one or more non-chiral compound of the formula I

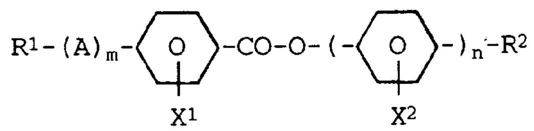

I

wherein

| | |
|---|---|
| $R^1$ and $R^2$ | are each independently a normal or branched alkyl or alkenyl residue with up to 16 C atoms wherein one or two nonadjacent $CH_2$ groups of these residues may be replaced by -O- or -CO- |
| A | denotes a group of the formula |

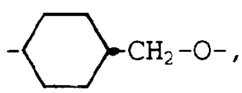, 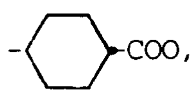 or 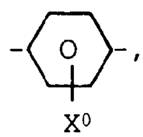,

| | |
|---|---|
| $X^0$, $X^1$ and $X^2$ | are each independently hydrogen or an halogen atom, |
| m | is 0 or 1, and |
| n | is 1 or 2, |

b) two, three or more chiral steroid ester of the formula II

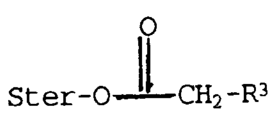

II

wherein

| | |
|---|---|
| $R^3$ | is a normal or branched alkyl or alkenyl residue with up to 16 C atoms wherein one $CH_2$ group may be replaced by -O-, -O-CO- or -CO-O-, and |
| Ster | denotes a saturated or unsaturated gonan-3-yl group being optionally substituted by up to 6 normal or branched alkyl residues with 1 to 10 C atoms, and |

c) at least one vehicle and, if desired, at least one auxiliary for decorative applications.

**5.** Composition according to Claim 4 characterized in that m is 0 and n is 1 in formula I.

**6.** Composition to Claim 4 or 5 characterized in that Ster denotes a cholesterin-4-yl group.

**7.** Compositions according to one of the Claims 4 to 6 characterized in that $R^3$ is an alkyl group with 2 to 10 C atoms.

**8.** Composition according to one of Claims 4 to 7 characterized in that the cholesteric liquid crystalline phases are microencapsulated before admixing to the vehicles.

## Patentansprüche

1. Verwendung einer dekorativen Zusammensetzung, bestehend aus einer Mischung aus:

   a) einer oder mehreren nichtchiralen Verbindungen der Formel I

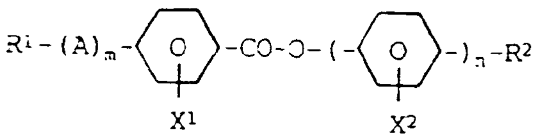

I

worin

| | |
|---|---|
| $R^1$ und $R^2$ | jeweils unabhängig einen normalen oder verzweigten Alkyl- oder Alkenylrest mit bis zu 16 C-Atomen, worin eine oder zwei nicht benachbarte $CH_2$-Gruppen dieser Reste auch durch -O- oder -CO- ersetzt sein können, |
| A | eine Gruppe der Formel |

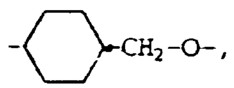

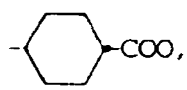

oder

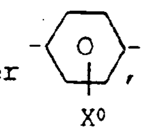

| | |
|---|---|
| $X^0$, $X^1$ und $X^2$ | jeweils unabhängig Wasserstoff oder ein Halogenatom, |
| m | 0 oder 1 und |
| n | 1 oder 2 bedeuten, |

   b) einem oder mehreren chiralen Steroidestern der Formal II

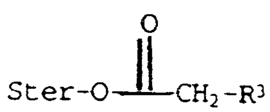

II

worin

| | |
|---|---|
| $R^3$ | einen normalen oder verzweigten Alkyl- oder Alkenylrest mit bis zu 16 C-Atomen, worin eine $CH_2$-Gruppe auch durch -O-, -O-CO- oder -CO-O- ersetzt sein kann, und |
| Ster | eine gesättigte oder ungesättigte, gegebenenfalls durch bis zu 6 normale oder verzweigte Alkylreste mit 1 bis 10 C-Atomen substituierte Gonan-3-ylgruppe bedeuten, sowie |

   c) mindestens einem Träger und gegebenenfalls mindestens einem Hilfsmittel,

   für dekorative Anwendungen.

2. Verwendung einer dekorativen Zusammensetzung nach Anspruche 1, dadurch gekennzeichnet, daß in Formel I m 0 und n 1 bedeuten.

3. Verwendung einer dekorativen Zusammensetzung nach Anspruch 1 oder 2, dadurch gekennzeichnet, daß Ster eine Cholesterin-4-ylgruppe bedeutet.

4. Dekorative Zusammensetzung, bestehend aus einer Mischung aus:

   a) einer oder mehreren nichtchiralen Verbindungen der Formel I

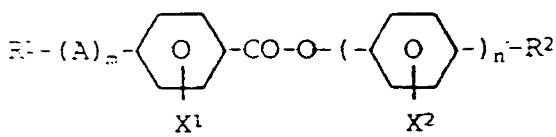

I

worin

| | |
|---|---|
| $R^1$ und $R^2$ | jeweils unabhängig einen normalen oder verzweigten Alkyl- oder Alkenylrest |

mit bis zu 16 C-Atomen, worin eine oder zwei nicht benachbarte $CH_2$-Gruppen dieser Reste auch durch -O- oder -CO- ersetzt sein können,

A eine Gruppe der Formel

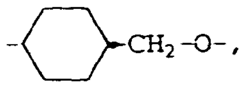 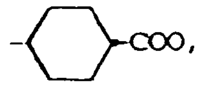 oder 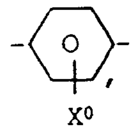

$X^0$, $X^1$ und $X^2$ jeweils unabhängig Wasserstoff oder ein Halogenatom,

m 0 oder 1 und

n 1 oder 2 bedeuten,

b) zwei, drei oder mehr chiralen Steroidestern der Formel II

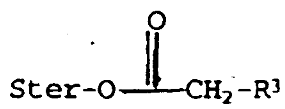

II

worin

$R^3$ einen normalen oder verzweigten Alkyl- oder Alkenylrest mit bis zu 16 C-Atomen, worin eine $CH_2$-Gruppe auch durch -O-, -O-CO- oder -CO-O- ersetzt sein kann, und

Ster eine gesättigte oder ungesättigte, gegebenenfalls durch bis zu 6 normale oder verzweigte Alkylreste mit 1 bis 10 C-Atomen substituierte Gonan-3-ylgruppe bedeuten, sowie

c) mindestens einem Träger und gegebenenfalls mindestens einem Hilfsmittel,

für dekorative Anwendungen.

**5.** Zusammensetzung nach Anspruch 4, dadurch gekennzeichnet, daß in Formel I m 0 und n 1 bedeuten.

**6.** Zusammensetzung nach Anspruch 4 oder 5, dadurch gekennzeichnet, daß Ster eine Cholesterin-4-ylgruppe bedeutet.

**7.** Zusammensetzung nach einem der Ansprüche 4 bis 6, dadurch gekennzeichnet, daß $R^3$ eine Alkylgruppe mit 2 bis 10 C-Atomen bedeutet.

**8.** Zusammensetzung nach einem der Ansprüche 4 bis 7, dadurch gekennzeichnet, daß die cholesterischen flüssigkristallinen Phasen vor der Vermischung mit den Trägerstoffen in Mikrokapseln eingeschlossen werden.

## Revendications

**1.** Utilisation d'une composition ornementale comprenant en mélange :

a) un ou plusieurs composé(s) non chiral(aux) répondant à la formule I :

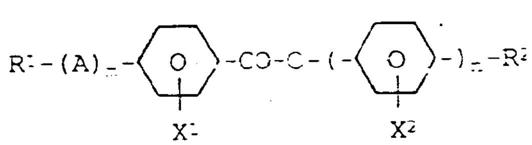

I

dans laquelle :

$R^1$ et $R^2$ représentent chacun indépendamment un reste alkyle ou alcényle normal ou ramifié contenant jusqu'à 16 atomes de carbone, un ou deux groupe(s) $CH_2$ non adjacent(s) de ces restes pouvant être remplacé(s) par -O- ou -CO-,

A représente un groupe de formule :

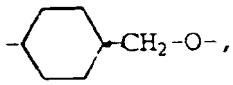, 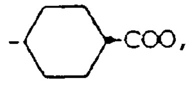, ou 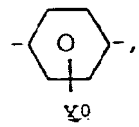,

$X^0$, $X^1$ et $X^2$ représentent chacun indépendamment un atome d'hydrogène ou d'halogène,
m est 0 ou 1, et
n est 1 ou 2,
b) un ou plusieurs ester(s) stéroidien(s) chiral(aux) de formule II

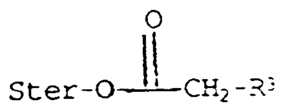 II

dans laquelle $R^3$ est un reste alkyle ou alcényle linéaire ou ramifié contenant jusqu'à 16 atomes de carbone, un groupe $CH_2$ pouvant être remplacé par -O-, -O-CO- ou -CO-O-, et
Ster désigne un groupe gonanyle-3 saturé ou non saturé et facultativement substitué par un maximum de 6 restes alkyle normal ou ramifié de 1 à 10 atomes de carbone, et
c) au moins un véhicule et, éventuellement, un agent auxiliaire,
pour des applications ornementales.

2. Utilisation de la composition ornementale selon la revendication 1, caractérisée en ce que dans la formule I, m est 0 et n est 1.

3. Utilisation de la composition ornementale selon la revendication 1 ou 2, caractérisée en ce que Ster désigne un groupe cholestérinyle-4.

4. Composition ornementale qui comprend en mélange :
a) un ou plusieurs composé(s) non chiral(aux) répondant à la formule I :

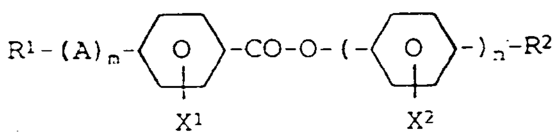 I

dans laquelle :
$R^1$ et $R^2$ représentent chacun indépendamment un reste alkyle ou alcényle normal ou ramifié contenant jusqu'à 16 atomes de carbone, un ou deux groupe(s) $CH_2$ non adjacent(s) de ces restes pouvant être remplacé(s) par -O- ou -CO-,
A représente un groupe de formule :

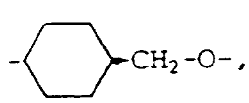, 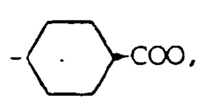, ou 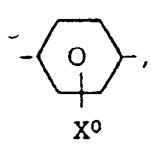,

$X^0$, $X^1$ et $X^2$ représentent chacun indépendamment un atome d'hydrogène ou d'halogène,
m est 0 ou 1, et
n est 1 ou 2,
b) deux, trois ou plusieurs ester(s) stéroidien(s) chiral (aux) de formule II

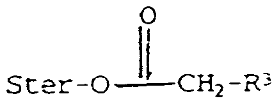

II

dans laquelle $R^3$ est un reste alkyle ou alcényle normal ou ramifié contenant jusqu'à 16 atomes de carbone, un groupe $CH_2$ pouvant être remplacé par -O-, -O-CO- ou -CO-O-, et
Ster désigne un groupe gonanyle-3 saturé ou non saturé et facultativement substitué par un maximum de 6 restes alkyle normal ou ramifié de 1 à 10 atomes de carbone, et
c) au moins un véhicule et, éventuellement, un agent auxiliaire,
pour des applications ornementales.

**5.** Composition selon la revendication 4, caractérisée en ce que dans la formule I, m est 0 et n est 1.

**6.** Composition selon la revendication 4 ou 5, caractérisée en ce que Ster désigne un grouse cholestérinyle-4.

**7.** Composition selon l'une quelconque des revendications 4 à 6, caractérisée en ce que $R^3$ est un radical alkyle de 2 à 10 atomes de carbone.

**8.** Composition selon l'une quelconque des revendications 4 à 7, caractérisée en ce qu'avant le mélange avec des véhicules, les phases cristallines liquides cholestériques sont soumises à un microenrobage.